# EUROPEAN PATENT SPECIFICATION

(11) **EP 0 910 428 B1**
(45) Date of publication and mention of the grant of the patent: **26.10.2005**
(21) Application number: 97916965.3
(22) Date of filing: 26.03.1997
(51) Int. Cl.: A61M 37/00, A61K 41/00, A61K 31/352, A61P 31/12

(54) **PHOTOPHERESIS TREATMENT OF CHRONIC HCV INFECTIONS**
PHOTOPHERESE-BEHANDLUNG VON HCV-INFEKTIONEN
TRAITEMENT PAR PHOTOPHERESE DES INFECTIONS CHRONIQUES DUES A L'HEPATITE C

(30) Priority: 29.03.1996 US 14612 P
(43) Date of publication of application: 28.04.1999
(73) Proprietor: THERAKOS, INC., Exton, PA 19341 (US)
(72) Inventor: McLAUGHLIN, Susan, N., Phoenixville, PA 19460 (US); STOUCH, Bruce, C., Newtown Square, PA 19073 (US); O'BRIEN, Christopher, B., Philadelphia, PA 19106 (US)
(74) Representative: Mercer, Christopher Paul
(86) International application number: PCT/US1997/004771
(87) International publication number: WO 1997/036634

(56) References cited:
- WO-A-97/21346
- WO-A-97/36581
- WO-A-97/37654
- US-A- 4 321 919
- US-A- 4 727 027
- US-A- 5 030 200
- US-A- 5 288 605
- DATABASE CHEMABS [Online] CHEMICAL ABSTRACTS SERVICE, COLUMBUS, OHIO, US; SARACENI, F.: "Evaluation of hepatitis B virus photoinactivation in serum and cellular blood components by the polymerase chain reaction" retrieved from STN Database accession no. 120:100747 XP002158404 & REPORT (1992), AFIT/CI/CIA-92-073;ORDER NO. AD-A254935, 66 PP. AVAIL.: NTIS FROM: GOV. REP. ANNOUNCE. INDEX (U. S.) 1993, 93(1), ABSTR. NO. 301,584,

## Description

### Background of the Invention

Extracorporeal photopheresis is a process where 8-Methoxypsoralen (8-MOP), a naturally occurring light-sensitive compound, is administered orally two hours prior to treatment; blood is then withdrawn from the patient, anticoagulated, and the white blood cells are separated by centrifugation and collected as a leukocyte enriched fraction. These 8-MOP containing leukocytes are then irradiated with ultraviolet A light (UVA) which binds the 8-MOP to pyrimidine bases in DNA and to intra- and extra-cellular proteins. These treated leukocytes are returned to the patient, and the result is an immunomodulation which has been found to be of clinical benefit in a number of disease states¹.

There are a number of diseases which are felt to primarily involve T-cells or are T-cell mediated. Diseases such as cutaneous T-cell lymphoma, organ allograft rejection after transplantation, and diseases such as progressive systemic sclerosis (PSS), rheumatoid arthritis and juvenile onset diabetes mellitus (JODM) are thought to be T-cell mediated.

Cutaneous T-cell lymphoma (CTCL) is a malignant disease that is progressive. Therapeutic options are limited. Edelson et al performed a multi-center trial² which showed that 24 of 29 (83%) of erythrodermic patients experienced a significant improvement in their disease. These positive responses were seen at a median time of 22.4 weeks after initiation of therapy. Of clinical significance, these patients were those whose diseases were resistant to prior therapy which is felt to be a poor prognostic group. In addition, a decrease in the amount of peripheral blood - involvement (Sezary cells) was seen. Actuarial data had indicated that median survival was increased to greater than 60 months from the onset of treatment in comparison with a historical median survival time of less than 30 months. In this original group of patients, remissions were sustained in eight of the subjects who were leukemic. Adverse reactions associated with photopheresis were rare.

Autoimmune diseases are characterized by a dysregulation of the immune system, characterized by specific cellular or humoral mediated destruction of specific organs or tissues in the patient. Examples of such diseases are rheumatoid arthritis and progressive systemic sclerosis.

Rheumatoid arthritis (R.A.) is an inflammatory disease that ultimately leads to joint destruction and is a generalized disease involving many organ systems. There are many agents in use for RA., however well tolerated agents with disease modifying potential are needed in as much as the disease is lifelong. In particular, a loss of efficacy and disease progression is seen in a high number of patients after starting secondary line therapy for R.A. Many of the second line agents are immunosuppressive and are themselves the reason for the major side effects such as infection. The need for development of a more specific, non-toxic immunomodulating therapy3 is great.

Progressive systemic sclerosis (PSS) is a connective tissue disease characterized by inflammatory and fibrotic changes in the skin and viscera. Treatment has been difficult. Anti-inflammatory drugs and corticosteroids are helpful in the early stages of the disease, but do not appear to influence the progression of the disease. Trials with D-penicillamine, methotrexate, cyclosporine, calcium channel blockers and prostagladins are underway, but these agents do not appear to influence the overall progression of the disease. As this disease has been considered to be T-cell mediated, Rock and colleagues have treated PSS patients with photopheresis4. In this trial, 56 patients were enrolled into a randomized non-blinded clinical trial. A significantly higher response rate was seen in the photopheresis treated group (68% response rate) compared to the D-penicillamine (control) group (32% response rate).

Juvenile onset diabetes mellitus (JODM) is felt to be mediated by the immune system resulting in the destruction of the cells in the pancreas responsible for the production of insulin. Patients with this disorder have not only dysregulation of their blood sugar levels, but the disease is characterized by a vasculopathy, resulting in specific organ damage leading to significant morbidity and mortality.
Other T-cell mediated phenomena include rejection of tissues that are foreign to the host. In the case of organ allograft transplantation, it is desirable to prevent this rejection with respect to the transplanted organ, however to otherwise maintain the competence of the immune system, in order to allow the body to combat infection and to allow other normal body defenses. The standard treatments after transplantation are limited as the immunosuppression regimens used to cause a state of general immunosuppression, which leads to the most common adverse reaction to this treatment, again infection, which may be bacterial or opportunistic infection. Immunomodulation which does not have immunosuppressive properties; this would be more desirable. Photopheresis has been shown to be effective; investigators at Loyola University have been able to successfully treat with photopheresis 13 of 14 cases of cardiac rejection refractory to standard immunosuppressive agents. In a variation of this situation, photopheresis has been successfully used (Rossetti et al⁵ and others) to treat successfully a patient with chronic graft versus host disease. This disorder is characterized by an introgenically induced immunoincompetent host, where immune competent cells (bone marrow or peripheral stem cells) are infused into a patient in such situations as treatment for various malignancies and leukemia. Here the transplanted immunocompetent cells attack the patient (the "host"). Here again, the issue is to modulate the immunocompetent cells without causing further immunosuppression and the side effects thereof

Photopheresis involves the extracorporeal exposure of peripheral blood leukocytes to 8-methoxypsoralen (8-MOP) photoactivated by ultraviolet A light, followed by the reinfusion of the treated white blood cells.

8-methoxypsoralen molecules in the blood enter the white blood cell nuclei and intercalate in the double-strand DNA helix. In an extracorporeal circuit, long wave ultraviolet light is directed at the leukocyte-enriched blood fraction within the UVAR Photopheresis System. The photoactivated drug, responding to the UVA energy, links to the thymidine base in the DNA helix. This results in cross-linking of thymidine bases which prevents the unwinding of the DNA during transcription. The plasma and altered leukocytes are then reinfused into the patient. The reinfusion of the photopheresis damaged leukocytes results in an delayed immune attack against these damaged leukocytes, as well as, otherwise unmodified WBC's displaying the same cell surface antigens.

### UVAR System

The treatment consists of three phases including 1) the collection of a buffy-coat fraction (leukocyte-enriched), 2) irradiation of the collected buffy coat fraction, and 3) reinfusion of the treated white blood cells. The collection phase has six cycles of blood withdrawal, centrifugation, and reinfusion steps. During each cycle, whole blood is centrifuged and separated in a pediatric pheresis bowl. From this separation, plasma (volume in each cycle is determined by the UVAR Instrument operator) and 40 ml buffy coat are saved in each collection cycle. The red cells and all additional plasma are reinfused to the patient before beginning the next collection cycle. Finally, a total of 240 ml of buffy coat and 300 ml of plasma and are separated and saved for UVA irradiation.

The irradiation of the leukocyte-enriched blood within the irradiation circuit begins during the buffy coat collection of the first collection cycle. The collected plasma and buffy coat are mixed with 200 ml of heparinized normal saline and 200 mg of UVADEX, (water soluble 8-methoxypsoralin). This mixture flows in a 1.4 mm thick layer through the PHOTOCEPTOR Photoactivation Chamber, which is inserted between two banks of UVA lamps of the PHOTOSETTE, PHOTOSETTE, UVA lamps irradiate both sides of this UVA-transparent PHOTOCEPTOR, chamber, permitting a 180-minute exposure to ultraviolet A light, yielding an average exposure per lymphocyte of 1-2 J/cm². The final buffy coat preparation contains an estimated 20% to 25% of the total peripheral blood mononuclear cell component and has a hematocrit from 2.5% to 7%. Following the photoactivation period, the volume is reinfused to the patient over a 30 to 45 minute period.

Systems employing these techniques are known whereby extracorporeal treatment of a patient's blood is undertaken. For example, in U.S. Patent No. 4,573,960--Goss, a patient is given a drug that requires photoactivation and the patient's blood is then withdrawn and separated into its components. The untreated components (red blood cells, some plasma, etc.) are returned to the patient. The patient is then disconnected from the treatment apparatus and the separated components, e.g., white blood cells, are exposed to ultraviolet light. Following photoactivation, the treated cells are returned to the patient.

In U.S. Pat. Nos. 4,321,919; 4,398,906; and 4,464,166, issued to Edelson, the external treatment methods for diseases in which there is a pathological increase of lymphocytes, such as cutaneous T-cell lymphoma, have been discussed. In these methods the patient's blood in the presence of a chemical or an antibody is irradiated with ultraviolet light. Ultraviolet light effects a bonding between the lymphocytes and the chemical or antibody thus inhibiting the metabolic processes of the lymphocytes.

A variety of human viruses are able to infect and replicate within mononuclear cells, or infectious viral particles may remain present within the mononuclear cells. The mononuclear cells can act as either a source for viral replication and spread of the virus, or as a reservoir of infectious virus particles which is difficult for the immune system to eliminate. Failure to eliminate these sources of infectious virus may lead to the establishment of a chronic condition. Viruses which can infect, replicate within, or reside in mononuclear cells include, but are not limited to, arthropod borne viruses, enteroviruses, paramyxoviruses (RSV), herpes viruses, cytomegalo-virus (CMV), Epstein-Barr virus (EBV), hepatitis B virus (HBV), hepatitis C virus (HCV), hepatitis D virus (HDV), hepatitis G virus (HGV), and HIV.

A variety of human non-viral pathogenic agents are able to infect and replicate within mononuclear cells, or the infectious non-viral pathogenic agents may remain present within the mononuclear cells. The mononuclear cells can act as either a source for replication and spread of the non-viral pathogenic agents, or as a reservoir of infectious non-viral pathogenic agents which is difficult for the immune system to eliminate. Failure to eliminate these sources of infectious non-viral pathogenic agents may lead to the establishment of a chronic condition. Non-viral pathogenic agents which can infect, replicate within, or reside in mononuclear cells include, but are not limited to, bacteria such as arthropod-borne bacteria, mycoplasma species, and mycobacteria species, and parasites such as plasmodium species and other arthropod-borne parasites.

Extracorporeal photopheresis has been successfully used to treat HIV infection (U.S. Patent No. 4,960,408) and psoralen compounds with long wavelength ultraviolet light has been shown to inactivate certain viruses in vitro, such as HIV (Quinnan, G.V. et al., 1986, Transfusion, 26, pp 481; Bisaccia, A. et al., 1990, Ann. Intern. Med., 113,. pp 270; Bisaccia, A. et al., 1991, Am. NY Acad. Sci., 636, pp 321), and influenza virus and herpes simplex virus (Redfield, D.C. et al., 1981; Infect. and Immun., 32, pp 1216). Bisaccia from Columbia University has studied ECP in a pilot trial as therapy for patients with AIDS-related complex. The rationale was that a combination of psoralen with UVA activation could damage HIV virus in vitro and that reinfusion of the damaged virus may initiate an immune response. The authors found that ECP produced an increase in the HIV-Ab production, increase in the CD8(+) lymphocytes, a decrease in the p24 antigen titer and the inability to culture HIV virus in 3 patients. Eleven of the 20 patients had improvement in their skin test antigen reactivities.

In addition, a reduced incidence of infection episodes was reported in patients receiving photopheresis treatment for immunosuppression following transplant surgery (Meiser, B.M. et al., 1994, Transplantation, 57, pp. 563). However, the results observed for the transplant surgery patients did not correlate with photopheresis treatment since infection episodes in general were recorded including patients who received a variety of treatments to prevent rejection of the transplanted organ.

### Summary of the Invention

The present invention is drawn to a method of treating chronic HCV infections using extracorporeal photopheresis. In particular, patients having chronic HCV infections are treated by the method of the present invention and the level or presence of HCV genetic material is reduced or undetectable in patients so treated. The method of the present invention involves the treatment of a patient's blood with a photoactivatable or photosensitive compound which is capable of binding to nucleic acids in infected nucleated cells upon activation of the compound by ultraviolet light. The photoactivatable or photosensitive compound may be administered to the patient's blood in vitro or in vivo by conventional administration techniques.

A portion of the patient's blood is then treated extracorporeally using photopheresis, which comprises subjecting the blood to ultraviolet light, preferably long wavelength ultraviolet light in the wavelength range of 320 to 400 nm, commonly called UVA light. The treated blood, or a fraction thereof, is returned to the patient following extracorporeal photopheresis to stimulate an immunological response by the patient's immune system against the infected cell population and/or against the HCV to inhibit progression of the viral infection. The viral genetic material is also damaged by this treatment, rendering the virus incapable of replication, thereby interrupting the spread of the virus and neutralizing infectious viral particles that reside in the cells.

### Brief Description Of The Drawings

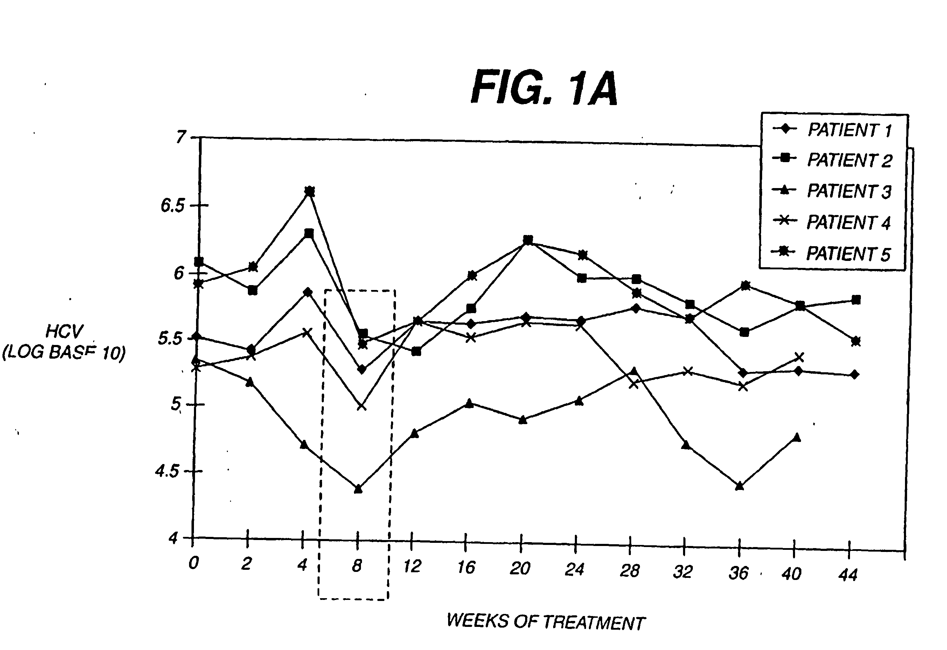

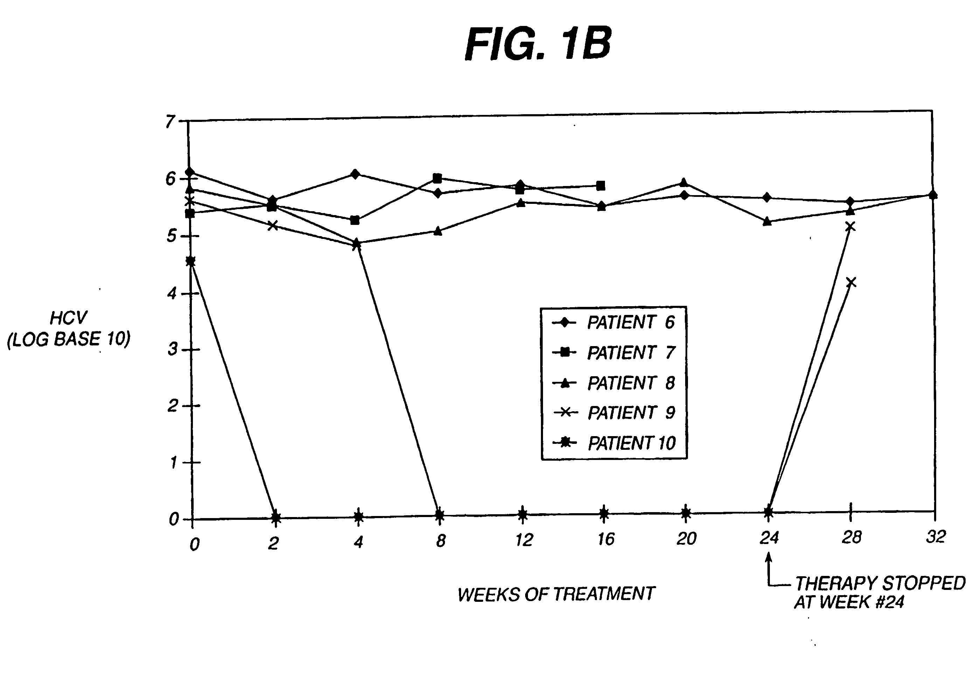

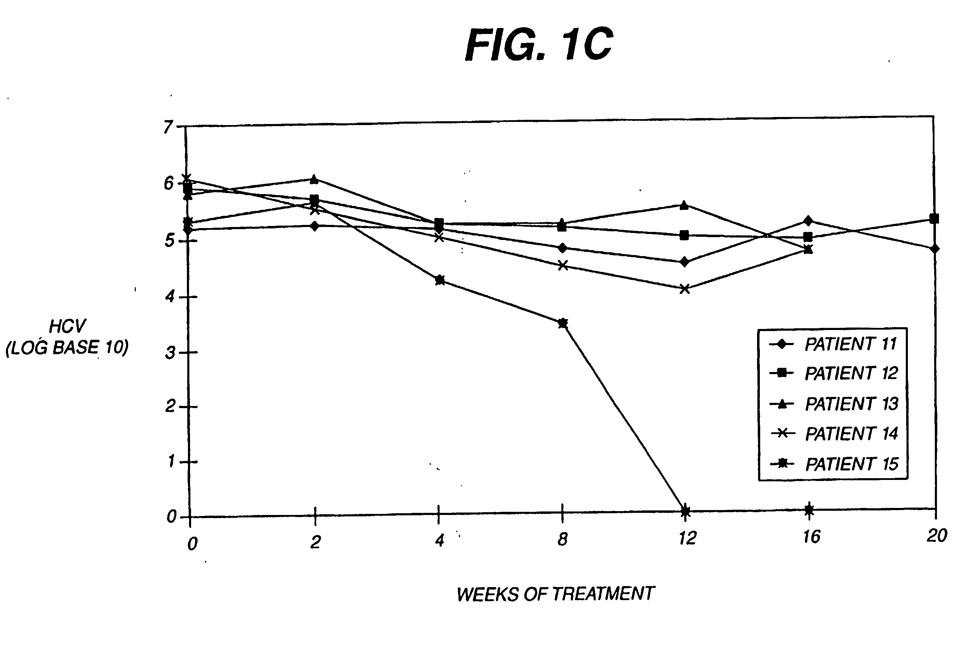

Figure 1, Panel A, Panel B, and Panel C show the patient results of treatment group 1, group 2, and group 3, respectively.

### Detailed Description of the Invention

The present invention is directed to the use of photopheresis to inactivate HCV and/or kill blood cells which have been infected with HCV. While it is not intended that the scope of the present invention be limited by any specific theory of operation, it is believed that HCV infections which are not controlled by the normal immunological response of a patient can be treated by damaging infected nucleated blood cells (such as mononuclear cells) using a photopheresis treatment according to the invention. The treated cells as well as killed and/or attenuated virus, peptides, native subunits of the virus itself (which are released upon cell break-up and/or shed into the blood) and/or pathogenic noninfectious viruses are then used to generate an immune response.

HCV is either able to infect and replicate within mononuclear cells, or infectious viral particles may remain present within the mononuclear cells. The mononuclear cells can act as either a source for viral replication and spread of the virus, or as a reservoir of infectious virus particles which is difficult for the immune system to eliminate. Failure to eliminate these sources of infectious virus may lead to the establishment of a chronic condition.

According to the claimed methods, a photoactivatable or photosensitive compound is first administered to the blood of a patient who is chronically infected with HCV. The photoactivatable or photosensitive compound may be administered in vivo (e.g. orally or intravenously) or may be administered in vitro to a portion of the patient's blood which has been removed from the patient by employing conventional blood withdrawal techniques.

In accordance with the present invention, the photoactivatable or photosensitive compound should be capable of binding to nucleic acids upon activation by exposure to electromagnetic radiation of a prescribed spectrum, e.g., ultraviolet light.

Next, the portion of the patient's blood to which the photoactive compound has been administered is treated by subjecting the portion of the blood to photopheresis using ultraviolet light. The photopheresis step is preferably carried out in vitro using an extracorporeal photopheresis apparatus. The photopheresis step in accordance with the present invention may also be carried out in vivo. A presently preferred extracorporeal photopheresis apparatus for use in the methods according to the invention is currently manufactured by Therakos, Inc., Westchester, Pa. under the name UVAR. A description of such an apparatus may be found in U.S. Patent No. 4,683,889, granted to R.L. Edelson on August 14, 1987. The exposure of blood to ultraviolet light in a photopheresis apparatus is within the ability of persons having ordinary skill in the art.

When the photopheresis step is carried out in vitro, at least a fraction of the treated blood is returned to the patient to increase the patient's immune response to the infected cell population and to the HCV itself Preferably, the treatment method described herein is repeated at an interval of about once per week to about once every four weeks. Preferred photoactive compounds for use in accordance with the present invention are compounds known as psoralens (or furocoumarins) which are described in U.S. Patent No. 4,321,919. Alternatively, the patient's blood can be separated on a standard photopheresis-type device and photoactivated on a separate device.

The preferred photoactivatable or photosensitive compounds for use in accordance with the present invention include the following:
psoralen; 8-methoxypsoralen; 4,5'8-trimethylpsoralen; 5-methoxypsoralen; 4-methylpsoralen; 4,4-dimethylpsoralen; 4-5'-dimethylpsoralen; 4'-aminomethyl-4,5',8-trimethylpsoralen; 4'-hydroxymethyl-4,5',8-trimethylpsoraten; and 4',8-methoxypsoralen. The most particularly preferred photosensitive compound for use in accordance with the invention is 8-methoxypsoralen.

The photosensitive compound, when administered to the patient's blood in vivo is preferably administered orally, but also and be administered intravenously and/or by other conventional administration routes.

The preferred oral dosage of the photosensitive compound is in the range of about 0.3 to about 0.7 mg/kg. most preferably about 0.6 mg/kg. When administered orally, the photosensitive compound should preferably be administered at least about one hour prior to the photopheresis treatment and no more than about three hours prior to the photopheresis treatment. If administered intravenously, the times would be shorter.

Alternatively the photosensitive compound may be administered to the patient's blood following its withdrawal from the patient, and prior to or contemporaneously with exposure to ultraviolet light. The photosensitive compound may be administered to whole blood or a fraction thereof provided that the target blood cells or blood components receive the photosensitive compound.

The photopheresis treatment in the treatment methods according to the invention is preferably carried out using long wavelength ultraviolet light (UVA) at a wavelength within the range of 320 to 400 nm. The exposure to ultraviolet light during the photopheresis treatment preferably has a duration of sufficient length to deliver about 1-2 J/cm² to the blood.

When the photopheresis treatment according to the invention is carried out in vivo, careful attention should be paid to controlling the maximum radiant exposure so as to avoid unnecessary injury to the patient. Methods for calculating maximum radiant exposure to ultraviolet light are known in the art.

The invention also provides methods for making vaccines against the HCV. According to the invention, a donor who is infected HCV may be utilized to produce a vaccine against his virus infection as follows.

First, a photosensitive compound as described hereinabove is administered to at least a portion of the donor's blood either prior to removal of the blood, either orally or intravenously, or after removal from the patient in which case it is administered in vitro. Optionally, a portion of the donor's blood could first be processed using known methods to substantially remove the erhythrocytes and the photoactive compound is then administered to the resulting enriched leukocyte fraction.

In any case, the portion of blood (or enriched leukocyte fraction thereof) to which the photosensitive compound has been administered is subjected to a photoactivation treatment using ultraviolet light, preferably UVA in the manner previously described. The treated blood or the treated enriched leukocyte fraction (as the case may be) is then administered back to the donor.

The following Examples are provided to illustrate the present invention and are not to be construed as a limitation thereon.

### Example 1

### Reduction of HCV in Chronic HCV Infected Patients

The fundamental defects that allows establishment of chronic hepatitis C following acute HCV infection are not known. Several investigators have suggested that impaired cellular immunity, either T cells or natural killer cells, may play a role. Weiner and coworkers from the Chiron Corporation have demonstrated the existence of a hypervariable region of the HCV genome in the E2/NS1 segment.⁶ This area codes for isolate-specific, B-cell antibody-binding linear epitopes that are expressed on the envelope surface of the HCV particle. The characteristics of this domain are similar to the V3 loop of the human immunodeficiency virus 1's gp 120 protein. The rapid mutation within this region may explain a loss of immune recognition and clearance of the hepatitis C virus.

Other authors postulate that there may be other possibilities for resistance to interferon treatment. There is evidence that the genotype of the virus may be just as important in influencing the outcome of therapy. It appears that some genotypes (subtypes) of the hepatitis C virus may be more resistant to therapy with interferon alpha than other types. Other investigators find a correlation to the pretreatment viral titer of the hepatitis C virus and the outcome after treatment with interferon alpha.

But, the basic immune mechanism which results in the clearance of the hepatitis C virus is still not known. Shirai from the National Cancer Institute has demonstrated that CD8(+) cytotoxic lymphocytes recognize a nonstructural protein with homology to RNA polymerase expressed in association with a HLA class I antigen on the surface of the hepatocytes.⁷ This results in the process of antibody-dependent cell-mediated cytotoxic destruction of the viral infected hepatocyte. This would be similar to the process used to clear the hepatitis B virus.

The hepatitis C virus is a positive-stand virus that replicates by producing a negative-strand RNA as a template. During active HCV viral replication, these negative-strand RNA templates are present in the patient's liver. Investigators have also found the presence of active, replicating hepatitis C viral particles in patient's peripheral blood mononuclear cells.⁸ Mononuclear cells, macrophages, T-cells and B-cells can be shown to contain negative-grand HCV RNA.

During therapy with interferon-alpha hepatitis C virus may disappear from the patient's liver and blood as measured by RT-PCR. Despite this apparent clearance of virus in some patients, there is very high (80-85%) relapse rate after interferon therapy. Investigators have demonstrated that the (few) patients who do not have HCV replication detected in the PBMCs at the end of therapy, do not relapse after interferon therapy and are apparent cures.^{9,10,11} It would appear that the mononuclear cell can serve as an immunologically protected site that shelters the hepatitis C from the immune system recognition and attack. Once therapy with interferon-alpha is withdrawn, the virus may leave the PBMCs and reinfect the patient's blood and the liver.

Ideal therapy would clear the hepatitis C virus from the liver, blood and infected mononuclear cells simultaneously. Extracorporeal photopheresis' mechanism of action causes an immunization against the abnormal T-cells rendering them more immunogenic. After ECP exposure, reinfusion of these altered T-cells causes a immunologic reaction that targets unirradiated T-cells carrying the same surface antigens.¹² This results in the production of a highly specific immune response against these abnormal cells (either the cancer clone, or perhaps T-cells which express viral antigens on their surface).

In addition, ideal therapy should be not be effected by the hepatitis C's hypervariable E2/NS1 1 region's tendency to mutate. This change in the viral particle envelope antigens causes an escape from the B-cell neutralizing antibodies. Photopheresis may also induce damage to the any free viral particle in the irradiated blood. This form of therapy would also target any newly formed hepatitis C escape mutants.

In 1986, Hoofnagle et al.¹³ published the first report of successful interferon-alpha therapy of chronic hepatitis C in an uncontrolled trial. Ten patients with chronic hepatitis C received subcutaneous injections of interferon for up to a year. Six patients still have normal LFTs and nondetectable HCV RNA by RT-PCR seven years later.

To date, interferon alpha-2b, recombinant (Intron A) is the only FDA approved treatment for chronic hepatitis in the United States. Davis et al. reported that a six month course of interferon alpha-2b at a dose of 3 million units three times a week, resulted in an 18% sustained response off treatment.¹⁴ Multiple other trials have reproduced this response rate. Biochemical relapse occurs in the majority of patients following withdrawal of therapy. ¹⁵ Detection of HCV RNA in serum by RT-PCR has been shown to correlate with normalization of serum transaminases on treatment and has also been shown to precede biochemical relapse once therapy is withdrawn. ^{16,17}

Researchers continue to devise new therapies to improve the low response rates. Higher interferon doses, ¹⁸ more prolonged treatment duration,¹⁹ different species of interferon,^{20,21} and adjuvant therapeutic phlebotomy to remove excess iron²² have all been tried with various degrees of success. Other forms of therapy are even less efficacious and are associated with frequent, side effects. Corticosteroids and acyclovir have been shown to have little benefit.

Fifteen (15) patients were randomized to either monotherapy with ECP alone (5 patients), or to combination therapy with ECP and interferon-alpha 3 MIU TIW (5 patients), or to ECP and interferon-alpha 6 MIU TIW (5 patients). All patients received ECP treatment in the Clinical Research Center (CRC) at the Hospital of the University of Pennsylvania.

### Group I (ECP alone)

Patients randomized to receive ECP alone had two treatments, on two consecutive days every two weeks. Patients received 24 ECP treatments over a 6 month period.

### Group II (ECP and IFN-( 3 MIU)

Three months after Group I starts ECP, patients randomized to receive ECP and interferon-alpha 3 MIU started therapy. Patients began with ECP treatments on two consecutive days every two weeks. Two weeks after the start of ECP, patients received interferon-alpha 3 MIU three times a week subcutaneously. If well tolerated patients will continue the ECP treatments for two consecutive days every two weeks for six additional months in combination with interferon-alpha. Patients received 24 ECP treatments over a 6 month period.

### Group III (PUVA and IFN-( 6 MIU)

Three months after Group II started ECP, patients randomized to receive ECP and interferon-alpha 6 MIU started therapy. Patients began with ECP treatments on two consecutive days every two weeks. Two weeks after the start of ECP, patients received interferon-alpha 6 MIU three times a week subcutaneously.

### Screening Period

Screening period measurements included a history and physical examination, symptom assessment, weight, and blood pressure. Laboratory tests include: complete blood count with differential, thyroid function tests, prothrombin time, chemistry panel and serum HCG (if indicated). Serum pregnancy HCG was be performed within 24 hours of the start of treatment. A complete blood count included hematocrit, hemoglobin, platelet count, and total WBC with differential including neutrophilis, lymphocytes, mononuclear cells, eosinophils, and basophils. A chemistry panel included total protein, albumin, total bilirubin, ALT, AST, alkalinine phosphatase, gamma GT, sodium potassium chloride, CO2 content, urea nitrogen, creatinine, glucose, calcium, phosphate, cholersterol, triglycerides and uric acid. Serum and peripheral blood mononuclear cells (PBMCs) were collected for determination of HCV-RNA titers by RT-PCR and for HCV genotype analysis. Serum from 7 ml of whole blood was collected and frozen at -70(C for future studies.

### Treatment Period

A symptom assessment, weight, blood pressure, complete blood count and ALT was performed at week 2 and every 4 weeks during treatment. HCV RNA titer in serum was monitored at week 2 and every four weeks during treatment. HCV RNA titer in PBMCs was measured at the end of treatment. Serum from 7 ml of whole blood was collected every eight (8) weeks and frozen at - 70(C for future studies. A follow-up history and physical examination, chemistry panel, thyroid function tests and prothrombin time was performed at the end of treatment.

### Follow-up Period

A symptom assessment, weight, blood pressure, complete blood count and serum HCV RNA was performed every 8 weeks during and at the last visit of the follow-up period. HCV RNA titer in PBMCs was measured at last follow-up visit. ALT was measured every four weeks and at last visit of follow-up. Serum from 7 ml of whole blood was collected every eight (8) weeks during follow-up and frozen at -70(C for future studies. A follow-up history and physical examination chemistry panel, thyroid function tests and prothrombin time, was be performed at the last visit in the follow-up period.

### Serum Collection

Blood was collected in serum separator (BD SST) tubes.
Within 2 hours of blood draw, the blood tubes were centrifuged at 1500 x g for 20 minutes. Serum was collected immediately and stored at -70°C.

### Mononuclear Cell Preparation

Blood was collected in a 8 ml capacity vacutainer CPT mononuclear cell preparation tube (Becton Dickinson). Tubes were centrifuged for 20 minutes at 1500 x g 18-25°C. The plasma was removed without disturbing the cell layer and frozen in 1 ml aliquots at -70°C. The cell layer was pipeted into a 15 ml conical tube and 10 ml of PBS (Phosphate Buffered Saline) was added. The tube was mixed gently 5 times and then centrifuged at 300 x g for 15 minutes. Once the cells were pelleted at the bottom of the tube, the supernatant was removed and discarded. The pellet was resuspended in 2 mls of PBS and then divided into 1 ml aliquots into 1.5 ml Sarstedt tubes. The Sarstedt tubes were spun in a microcentrifuge on high for 10 minutes. The supernatant was removed and stored at -70(C until PCR analysis was performed. PCR analysis was performed using the Amplicor assay (see below).

### Amplicor Assay for Serum or Mononuclear Cell HCV

The HCV RNA assay is a prototype PCR-based assay using a single primer pair (biotinylated downstream primer) from the 5'-untranslated region of HCV. Uracil-N-glycosylase was incorporated to prevent amplicon contamination. The HCV target RNA was reverse transcribed and amplified in a single tube reaction using the enzyme rTth DNA polymerase in the presence of Mn++. After amplification, the products were alkaline denatured and hybridized to a HCV specific probe. Detection was carried out in a 96 microwell format using avidin-horse radish peroxidase. The result of the assay was expressed in A450 units as detected on a standard microwell plate reader.

### Efficacy Criteria

Response to treatment was measured using the following virologic and biochemical criteria:

### Virologic Criteria (Prime Criteria)

HCV-RNA in serum was monitored pre-study, during therapy to determine response, and during the 24 week follow-up period to determine stability of the response. Response to treatment was scored according to the following criteria:
a) No response: HCV RNA remains detectable by PCR during the treatment and follow-up periods.
b) Complete Response: HCV RNA is nondetectable by PCR on therapy at the end of the treatment period and becomes detectable before end of the follow-up period.
c) Cure: HCV RNA by PCR is nondetectable at the end of the treatment period and remains nondetectable at the end of the follow-up period.

Results of this study show that photopheresis alone or in conjunction with interferon treatment substantially reduces the level of HCV genetic material.

The use of photopheresis alone reduced the virus level in chronic HCV patients who have previously failed other treatments. Table 1 (and Figure 1 Panel A) depicts the HCV level of each patient at baseline and after 8 weeks on therapy (boxed region of Figure 1, Panel A).

The combination of photopheresis and interferon-α eliminates the presence of virus, as detected by RT-PCR, in the peripheral blood of chronically infected HCV patients. Figures 1B and 1C depict the HCV levels for the patients before, during and after the completion of treatment. The treatment effect was lost after the photopheresis was discontinued.

| **Hepatitis C** | | |
|---|---|---|
| **Group 1: Photopheresis Only** | | |
| | **HCV (Log Base 10)** | |
| **Patient Number** | **Week 0 of Treatment** | **Week 8 of Treatment** |
| 1 | 5.51 | 5.27 |
| 2 | 6.07 | 5.54 |
| 3 | 5.32 | 4.39 |
| 4 | 5.27 | 5.00 |
| 5 | 5.91 | 5.47 |

### Bibliography

1. Edelson RL. Photopheresis: a clinically relevant immunobiologic response modifier. [Review] *Ann NY Acad Sci*. 191; 636:154-64.
2. Edelson R, Berger C, Gasparro F. et al. Treatment of cutaneous T cell lymphoma by extracorporeal photochemotherapy: Preliminary results. *N Engl J Med* 1987;316:297-303.
3. Malawista S, Trock D, Edelson R. Treatment of rheumatoid arthritis by extracorporeal photochemotherapy: a pilot study. *Arthritis Rheum* 1991;34:646-54.
4. Rook AH, Freundlich B, Jegasothy BV, et al. Treatment of systemic sclerosis with extracorporeal photochemotherapy: Results of a multicenter trial. *Arch Dermatol* 1992;128:337-46.
5. Rossetti et al., 1995, Transplant, 59:1, pp.149-151.
6. Weiner AJ, Geysen HM, Christopherson C, Hall JE, Mason TJ, Saracco G, et al. Evidence for immune selection of hepatitis C virus (HCV) putative envelope glycoprotein variants: Potential role of chronic HCV infections. *Proc Natl Acad Sci* 1992; 89:3468-72.
7. Shirai M, Akatsuka T, Pendleton CD, Houghten R, Wychowski C, Mihalik K, et al. Induction of cytotoxic T cells to a cross-reactive epitope in the hepatitis C virus nonstructural RNA polymerase-like protein. *J Virology* 1992; 66:4098-106.
8. Zignego AL, Macchia D, Monti M, Thiers V, Mazzetti M, Foschi M, et al. Infection of peripheral mononuclear blood cells by hepatitis C virus. *J Hepatology* 1992; 15:382-6.
9. Quian C, Camps J, Maluenda MD, Civeira MP, Prieto J. Replication of hepatitis C virus in peripheral blood mononuclear cells: Effect of alpha-interferon therapy. *J of Hepatology* 1992;6:380-3.
10. Cambell C, Dailley PJ, Urdea MS, Wilber J, Collins MA, Mason AL, et al. HCV RNA in peripheral blood mononuclear cells: Effect of alpha-interferon therapy. *J of Hepatology* 1992; 16:380-3.
11. Gil B, Qian C, Riezu-Boj JI, Civeira MP, Prieto J. Hepatic and extrahepatic HCV RNA strands in chronic hepatitis C: different patterns of response to interferon treatment. *Hepatology* 1993; 18:1050-4.
12. Edelson RL. Photopheresis: a clinically relevant immunobiologic response modifier. [Review] *Ann NY Acad Sci*. 191; 636:154-64.
13. Hoofnagle JH, et al. Treatment of chronic non-A, non-B hepatitis with recombinant human alpha interferon: A preliminary report. *N Eng J Med* 1986, 315:1575.
14. Davis GL, Balart LA, Schiff ER, Lindsay K, Bodenheimer HC, Perillo RP, Carey W, et al. Treatment of chronic hepatitis C with recombinant interferon alfa: a multicenter randomized, controlled trial. *N Engl J Med* 1989;321:1501-1506.
15. DiBisceglie AM, Martin P, Kassianides C, Lisker-Melman M, Murray L, Waggoner J, Goodman A, et al. Recombinant interferon alpha therapy for chronic hepatitis C: A randomized, double blind, placebo-controlled trial. *N Eng J Med* 1989; 321: 1506-1510.
16. Farci P, Alter HJ, Wong D, Miller RH, Shih JW, Jett B, Purcell RH. A long term study of hepatitis C virus replication in non-A, non-B hepatitis. *N Engl J Med* 1991; 325:98-104.
17. Shindo M, Dibisceglie AM, Cheung L, Shih JW, Cristiano K, Feinstone SM, Cristiano K, Hoofnagle JH. Decrease in serum hepatitis C viral RNA during alpha-interferon therapy for chronic hepatitis. *Ann Intern Med* 1991; 115:700-704.
18. Watson, AR, Bartlome P. High-dose interferon alfa-2A for the treatment of chronic hepatitis C. [Review] *Ann Pharmacotherapy* 1994; 28:341-2.
19. Gomez-Rubio M. Porres JC, Castillo I, Quiroga JA, Moreno A, Carreno V. Prolonged treatment (18 months) of chronic hepatitis C with recombinant alpha-interferon in comparison with a control group. *J Hepatology* 1990;11:S63-7.
20. Saez-Royuela F, Porres JC, Moreno A, Castillo I, Martinez G, Galiana F, et al. High doses of recombinant alpha-interferon or gamma-interferon for chronic hepatitis C: a randomized, controlled trial. *Hepatology* 1991; 13:327-31.
21. Nakano Y, Kiyosawa K, Sodeyama T, Tanaka E. Comparative study of clinical, histological and immunological responses to interferon therapy in type non-A, non-B and type B chronic hepatitis. *Am J Gastroenterol* 1990;85:24-9.
22. Hayashi H, Takikawa T, Nishimura N, Yano M, Isomura T, Sakamoto N. Improvement of serum aminotransferase levels after phlebotomy in patients with chronic active hepatitis C and excess hepatic iron. *Am J Gastroenterol* 1994; 89:970-3.

## Claims

1. Use of a photoactivatable compound in the manufacture of a medicament for the treatment of a patient having a chronic HCV infection by exposing said patient's blood containing said photoactivatable compound to light of a wavelength which activates said photoactivatable compound.

2. Use of a photoactivatable compound and interferon alpha in the manufacture of a medicament for the sequential treatment of a patient having chronic HCV infection by exposing said patient's blood containing said photoactivatable compound to light of a wavelength which activates said photoactivatable compound.

3. The use of claim 2, wherein the use of the photoactivatable compound is on two consecutive days every two weeks.

4. The use of claim 3, wherein the use of the photactivatable compound is over a 3 month period.

5. The use of claim 2, wherein said treatment with interferon alpha begins two weeks after the use of the photoactivatable compound.

6. The use of claim 6, wherein said treatment with interferon alpha occurs three times a week.

7. The use of any one of claims 1 to 6, wherein said photoactivatable compound is a psoralen.

8. The use of claim 7, wherein said psoralen is 8-methoxypsoralen.

## Patentansprüche

1. Verwendung einer photoaktivierbaren Verbindung bei der Herstellung eines Medikaments für die Behandlung eines Patienten mit einer chronischen HCV-Infektion durch Exponieren von Blut des Patienten, das die photoaktivierbare Verbindung enthält, gegenüber Licht einer Wellenlänge, das die photoaktivierbare Verbindung aktiviert.

2. Verwendung einer photoaktivierbaren Verbindung und Interferon alpha bei der Herstellung eines Medikaments für die sequentielle Behandlung eines Patienten mit chronischer HCV-Infektion durch Exponieren von Blut des Patienten, das die photoaktivierbare Verbindung enthält, gegenüber Licht einer Wellenlänge, das die photoaktivierbare Verbindung aktiviert.

3. Verwendung nach Anspruch 2, wobei die Verwendung der photoaktivierbaren Verbindung an zwei aufeinanderfolgenden Tagen alle zwei Wochen ist.

4. Verwendung nach Anspruch 3, wobei die Verwendung der photoaktivierbaren Verbindung über eine Dauer von drei Monaten ist.

5. Verwendung nach Anspruch 2, wobei die Behandlung mit Interferon alpha zwei Wochen nach der Verwendung der photoaktivierbaren Verbindung beginnt.

6. Verwendung nach Anspruch 6, wobei die Behandlung mit Interferon alpha dreimal pro Woche stattfindet.

7. Verwendung nach einem der Ansprüche 1 bis 6, wobei die photoaktivierbare Verbindung ein Psoralen ist.

8. Verwendung nach Anspruch 7, wobei das Psoralen 8-Methoxypsoralen ist.

## Revendications

1. Utilisation d'un composé photo-activable dans la fabrication d'un médicament pour le traitement d'un patient atteint d'une infection chronique par le VHC par exposition du sang dudit patient contenant ledit composé photo-activable à une lumière d'une longueur d'onde activant ledit composé photoactivable.

2. Utilisation d'un composé photo-activable et de l'interféron α dans la fabrication d'un médicament pour le traitement séquentiel d'un patient atteint d'une infection chronique par le VHC par exposition du sang dudit patient contenant ledit composé photo-activable à une lumière d'une longueur d'onde activant ledit composé photoactivable.

3. Utilisation selon la revendication 2, dans laquelle on utilise le composé photo-activable deux jours consécutifs toutes les deux semaines.

4. Utilisation selon la revendication 3, dans laquelle on utilise le composé photo-activable pendant une période de trois mois.

5. Utilisation selon la revendication 2, dans laquelle ledit traitement par l'interféron α commence deux semaines après l'utilisation du composé photo-activable.

6. Utilisation selon la revendication 5, dans laquelle ledit traitement par l'interféron α est administré trois fois par semaine.

7. Utilisation selon l'une quelconque des revendications 1 à 6, dans laquelle ledit composé photo-activable est un psoralène.

8. Utilisation selon la revendication 7, dans laquelle ledit psoralène est le 8-méthoxypsoralène.
